(19) Europäisches Patentamt

European Patent Office

Office européen des brevets

(11) Veröffentlichungsnummer: **0 408 496 A2**

(12) **EUROPÄISCHE PATENTANMELDUNG**

(21) Anmeldenummer: 90810498.7

(22) Anmeldetag: 03.07.90

(51) Int. Cl.5: **A61K 9/20**, A61K 9/22, A61K 9/36, A61K 9/32, A61K 9/48

(30) Priorität: 12.07.89 CH 2616/89

(43) Veröffentlichungstag der Anmeldung: 16.01.91 Patentblatt 91/03

(84) Benannte Vertragsstaaten: AT BE CH DE DK ES FR GB GR IT LI LU NL SE

(71) Anmelder: CIBA-GEIGY AG Klybeckstrasse 141 CH-4002 Basel(CH)

(72) Erfinder: Khanna, Satish C., Dr. Rütistrasse 26 CH-4103 Bottmingen(CH)

(54) **Feste Darreichungsform für pharmazeutische Wirkstoffe.**

(57) Gegenstand der Erfindung ist eine feste pharmazeutische Darreichungsform aus der der aktive Wirkstoff bzw. das Wirkstoffgemisch annähernd pH-unabhängig, d.h. unabhängig von den Wasserstoffionen-und Hydroxylionenkonzentrationen und/oder anderer Ionen und auch unabhängig von Enzymen der umgebenden Flüssigkeit, gezielt nach einer bestimmten Zeit, d.h. nach Ablauf eines bestimmten Zeitintervalles quantitativ nach Freigabe sofort oder kontinuierlich in Lösung gehen kann, dadurch gekennzeichnet, dass die einen Wirkstoff oder ein Wirkstoffgemisch, einen in Wasser quellbaren nicht-kolloidalen Hilfsstoff (Sprengmittel) und mindestens einen in Wasser löslichen Trägerstoff (Osmose induzierenden Stoff) und gegebenenfalls anderer Hilfsstoffe enthaltende Darreichungsform mit einer semipermeablen Membran überzogen wird, die beim Eindringen von wässriger Flüssigkeit durch den Quelldruck (Sprengen) des quellbaren nichtkolloidalen Hilfsstoffes und durch den gleichzeitig auftretenden osmotischen Druck und gegebenenfalls intern erzeugten Gasdruck nach einer bestimmten Zeit lidartig aufspringt und den Wirkstoff bzw. das Wirkstoffgemisch quantitativ freigibt. Ferner ein Verfahren zur Herstellung und die Verwendung der festen pharmazeutischen Darreichungsform.

EP 0 408 496 A2

## FESTE DARREICHUNGSFORM FÜR PHARMAZEUTISCHE WIRKSTOFFE

Gegenstand der Erfindung ist eine feste Darreichungsform, die geeignet ist, den pharmazeutischen Wirkstoff bzw. Wirkstoffgemisch annähernd pH-unabhängig, d.h. unabhängig von den Wasserstoffionen-, Hydroxylionen-Konzentrationen und/oder anderer Ionen, wie z.B. Phosphationen und auch Enzymen der umgebenden Körperflüssigkeit, gezielt nach einer bestimmten Zeit, d.h. nach Ablauf eines bestimmten Zeitintervalls in diese quantitativ abgeben kann.

Die an sich älteste und bekannteste Art der Verabreichung eines Medikamentes, die der oralen Verabreichung, wird auch heute noch als äusserst zweckdienlich erachtet, um den pharmazeutischen Wirkstoff durch sofortige Freigabe, beispielsweise sofortige Auflösung über den Gastrointestinaltrakt in die systemische Blutzirkulation des Körpers einzubringen. Um die therapeutische Wirksamkeit jedoch zu garantieren, müssten diese pharmazeutischen Darreichungsformen jedoch wiederholt mehrmals in bestimmten Zeitintervallen am Tage eingenommen werden. In der Folge wurden auch weitere peroral zu verabreichende pharmazeutische Darreichungsformen mit verzögerter oder auch wiederholter Wirkstofffreigabe, sogenannte Retardformen (slow release- und sustained release-Formen) und auch Mehrfachformen (Repeatformen) entwickelt, die eine möglichst konstante, kontinuierliche und langandauernde Wirkstoffkonzentration oder auch langandauernde wechselhafte Wirkstoffkonzentration im Blutkreislauf ermöglichen, wobei die erzielte Wirkstoffkonzentration jedoch von der physiologischen Beschaffenheit der gastrointestinalen Flüssigkeiten direkt abhängig war.

Die entwickelten Retard- bzw. Repeatformen lassen durchaus eine kontinuierliche Freigabe des Wirkstoffes in konstanter bzw. wechselhafter (alternierender) Konzentration zu, jedoch konnte bisher das Bedürfnis einer gezielten, plötzlichen, quantitativen Freigabe nach einer bestimmten Zeit, d.h. nach Ablauf eines Zeitintervalles, im einfachen oder auch wiederholten Falle nicht erfüllt werden, wobei die zu erzielende Freigabe auch unabhängig vom pH, d.h. unabhängig von der Wasserstoffionen- und Hydroxylionenkonzentration und/oder auch anderen Ionen und Enzyme verlaufen sollte.

Es wird von Lachman et al. in "Theory and Practice of Industrial Pharmacy", Seiten 430-465 (herausgegeben von Lea und Febinger, Philadelphia, Pennsylvania, 1976), festgestellt, dass die Wirkstoffabgabe der beschriebenen Darreichungsformen (Retard- und Repeat-formen) und auch anderer konventioneller Darreichungsformen von der Löslichkeit des Wirkstoffes und diese wiederum vom pH-Wert der umgebenden Körperflüssigkeit abhängig ist.

Es ist bekannt, dass die Löslichkeit vieler pharmazeutischer Wirkstoffe aus den bisher bekannten Darreichungsformen inklusiv Retard- und Repeatformen, in diesen pH-Bereichen sehr verschieden sind. Die Wirkstoffe können je nach ihrer Löslichkeit in verschiedene Klassen eingeteilt werden, beispielsweise in die Klasse der Wirkstoffe, die sich durch schlechte Löslichkeit oder Unlöslichkeit im niederen pH-Bereich des Magens und grössere Löslichkeit im höheren pH-Bereich des Intestinaltrakts auszeichnen. Eine andere Klasse an Wirkstoffen charakterisiert sich durch Löslichkeit im niederen pH-Bereich und Unlöslichkeit oder schlechtere Löslichkeit im höheren pH-Bereich.

Ferner gibt es jedoch auch Wirkstoffe, deren Löslichkeit durch Fremddionen, beispielsweise Wasserstoff- und Phosphationen, stark beeinflusst werden. Durch Fremddionen können Aussalzeffekte oder auch die Ausfällung schwer löslicher Salze hervorgerufen werden, so dass eine vollständige Freigabe des in der Darreichungsform enthaltenden Wirkstoffes nicht stattfinden kann.

Bekannt ist aus der DOS 1617724 eine mit einem Film überzogene Darreichungsform, die nach vorgegebener Zeit durch entstehenden Riss bzw. Platzen der Membran den Wirkstoff, bzw. das Wirkstoffgemisch zeitlich gesteuert freigeben kann. Das Platzen der Membran wird durch den Druck des kolloidalen Quellmittels nach dem Eindringen von Körperflüssigkeit verursacht, wobei entweder verhältnismässig gut oder auch verhältnismässig schlecht in den physiologische Flüssigkeiten lösliche Wirkstoffe verwendet werden können. Gegebenenfalls werden zusätzlich auch Weichmacher, wie z.B. Glycerin hinzugegeben, um das Quellen der kolloidalen Quellmittel, beispielsweise Gelatine, zu erleichtern. Die in der oben angegebenen DOS 1617724 beschriebene Darreichungsform weist jedoch den Nachteil auf, dass durch den Riss der Membran nicht die gesamte Wirkstoffmenge sofort verfügbar ist, da nach erfolgtem Riss der Membran diese den Grossteil des Wirkstoffes noch weiterhin umhüllt und nicht sofort freigibt. Ueberraschenderweise kann dieser Nachteil jedoch ausgeräumt werden, in dem man erfindungsgemäss zusätzlich zum Quelldruck noch einen durch Osmose erzeugten Druck gleichzeitig auf die Membran einwirken lässt, wobei diese sich lidartig, vergleichsweise wie eine Muschel, öffnet und der Wirkstoff quantitativ sofort für die Freigabe zur Verfügung steht, wobei er sofort oder, wenn in retardierter Form vorliegend, auch kontinuierlich in Lösung gehen kann. Gegebenenfalls kann der erfindungsgemäss verwendete Osmosedruck noch durch einen intern erzeugten Gasdruck aus freigesetztem Kohlendioxyd erhöht werden, wenn man als Osmose induzierende Trägerstoffe Salze der Kohlensäure, z.B. Alkali- oder auch Erdalkalicarbonate oder auch -hydrogencarbonate in Gegenwart einer wasserlöslichen organischen Säure, wie z.B. Zitronensäure oder auch Weinsäure, beide als Osmose induzierenden Trägerstoffe verwendet. Beim Hineindiffundieren von Wasser der vorhandenen wässrigen Körperflüssigkeit in den Wirkstoff enthaltenden Kern wird durch die Reaktion der obengenannten Hilfsstoffe untereinander Kohlendioxyd freigesetzt und hiermit neben dem osmotischen Druck und Quelldruck noch ein zusätzlicher mechanischer Druck durch den internen Gasdruck erzeugt, der auf die Membran einwirkt.

Die erfindungsgemässe feste Darreichungsform, aus der der aktive Wirkstoff bzw. das Wirkstoffgemisch annähernd pH-unabhängig, d.h. unabhängig von den Wasserstoffionen- und Hydroxylionenkonzentrationen und/oder anderer Ionen und auch unabhängig von Enzymen der umgebenden Flüssigkeit, gezielt nach einer bestimmten Zeit, d.h. nach Ablauf eines bestimmten Zeitintervalles quantitativ sofort oder kontinuierlich in Lösung gehen kann, ist dadurch gekennzeichnet, dass die einen Wirkstoff oder ein Wirkstoffgemisch, einen nicht-kolloidalen in Wasser quellbaren Hilfsstoff (Sprengmittel) und mindestens einen in Wasser löslichen Trägerstoff (Osmose induzierenden Stoff) und gegebenenfalls andere Hilfsstoffe enthaltende Darreichungsform mit einer semipermeablen Membran überzogen wird, die beim Eindringen von Wasser der vorhandenen Körperflüssigkeit durch den Quelldruck des quellbaren nicht-kolloidalen Hilfsstoffes und durch den gleichzeitig auftretenden osmotischen Druck und gegebenenfalls intern erzeugten Gasdruck nach einer bestimmten, vorgegebenen Zeit lidartig aufspringt und den Wirkstoff bzw. das Wirkstoffgemisch quantitativ freigibt.

Der Wirkstoff bzw. das Wirkstoffgemisch kann je nach Lösungsvermögen sofort oder, wenn in Retardform gegeben auch kontinuierlich in Lösung gehen.

Die erfindungsgemässe Darreichungsform besteht im wesentlichen aus einer Filmtablette (Pressling) oder einem Pellet mit einem osmotisch aktiven Kern, der neben dem Wirkstoff bzw. Wirkstoffgemisch und dem nichtkolloidalen quellbaren Hilfsstoff auch mindestens einen wasserlöslichen Trägerstoff (Osmose induzierenden Stoff) und andere Hilfsstoffe, wie z.B. Gleitmittel und Retardierungsmittel enthalten kann und einer semipermeablen Membran als Filmüberzug. Wie bereits oben gesagt, können die wasserlöslichen Trägerstoffe Salze der Kohlensäure, wie z.B. Alkali- oder auch Erdalkalicarbonate oder -hydrogencarbonate und auch organische wasserlösliche Säuren, wie z.B. die Zitronensäure umfassen. Die Darreichungsform kann jedoch beispielsweise auch aus einer Kapsel, wie z.B. aus einer Gelatinekapsel bestehen, die den Wirkstoff bzw. das Wirkstoffgemisch, den quellbaren Hilfsstoff, mindestens einen wasserlöslichen Trägerstoff und andere Hilfsstoffe, wie z.B: Gleitmittel und Retardierungsmittel pulverförmig enthält und mit einer semipermeablen Membran als Film überzogen ist.

Durch die semipermeable Membran wird Wasser mit einer bestimmten Geschwindigkeit angezogen, die durch die Zusammensetzung und Dicke der Membran gesteuert werden kann. Das in den Kern eingedrungene Wasser der vorhandenen Körperflüssigkeit löst den Osmose induzierenden Trägerstoff und gegebenenfalls den Wirkstoff. Dabei entsteht ein osmotischer Druck, der umso grösser ist, je mehr Moleküle bzw. Ionen in Lösung gehen, wobei im Normalfall annähernd eine gesättigte Lösung entsteht.

Im einfachsten Fall kann beim Eindringen des Wassers der osmotische Druck, der als Folgereaktion auch das Quellen des quellbaren Hilfsstoffes hervorruft, teilweise durch den Wirkstoff bzw. durch das Wirkstoffgemisch selbst erzeugt werden, der jedoch für sich alleine genommen nicht gross genug ist, um das Platzen der Membran in gewollter Weise hervorzurufen, so dass ein Zusatz mindestens eines Osmose erzeugenden löslichen Trägerstoffs unumgänglich ist. Sollte der Wirkstoff jedoch in Wasser schwer oder gar unlöslich sein, so muss ein grösserer Anteil eines in Wasser löslichen Trägerstoff zugegeben werden, um den notwendigen osmotischen Druck zu erzeugen. Hiermit kann der für die Induktion des arbeitenden Prinzipes der Darreichungsform notwendige osmotische Druck erzielt werden, so dass das zum Ausgleich des osmotischen Gefälles eintretende Wasser die gewünschte Quellung des quellbaren nicht-kolloidalen Hilfsstoffes (Sprengmittels) hervorruft und nach einer bestimmten Zeit, d.h. nach Ablauf eines bestimmten Zeitintervalles, die lidartige Oeffnung (Sprengung) der Membran, hervorgerufen durch den Quell- und osmotischen Druck und gegebenenfalls intern erzeugten Gasdruck für die Freigabe des Wirkstoffes erfolgt.

Durch die geeignete Semipermeabilität der Membran und durch den Zusatz eines wasserlöslichen Trägerstoffes im Kern der Tablette, kann die erfindungsgemässe Darreichungsform annähernd als pH-unabhängig, d.h. von der Wasserstoffionen- und Hydroxylionenkonzentration und/oder anderen Ionen, wie z.B. Phosphationen und auch Enzymen, beispielsweise im Verdauungstrakt, hergestellt werden. Im Extremfalle kann die erfindungsgemässe feste, beispielsweise peroral zu verabreichende Darreichungsform auch für Wirkstoffe, bzw. Wirkstoffgemische verwendet werden, die in einem bestimmten pH-Bereich des Gastrointestinaltraktes schwer oder gar unlöslich sind.

Die Zeit der Freigabe des Wirkstoffes kann

a) durch die Wasserdurchlässigkeit des Membranfilmes,

b) durch die Filmdicke,

c) durch die mechanische Stärke, d.h. Elastizität und Reissfestigkeit der Membran und

d) durch die Quelleigenschaften des sich im Kern befindenden quellbaren Hilfsstoffes und durch die Fähigkeit des Trägerstoffes, einen osmotischen Druck durch Auflösung zu induzieren und

e) durch die Gesamtoberfläche der Darreichungsform bestimmt werden. Durch Variation der verschiedenen Freiheitsgrade a), b), c), d) und e) kann die Zeit der Freigabe (Zeitbombe) präzise manipuliert werden.

Im Grunde genommen eignen sich alle in der Literatur bekannten semipermeablen Membranen, die wasserdurchlässige Eigenschaften aufweisen, für die Herstellung des Filmes der erfindungsgemässen Darreichungsform.

Als semipermeable Membranen für die Filmschicht eignen sich die in der Literatur, beispielsweise im US Patent Nr. 3 916 889 und Nr. 3 977 404 beschriebenen semipermeablen Membranen, die für den Durchgang von Wasser aus der vorhandenen Körperflüssigkeit und nicht des gelösten Wirkstoffes und hiermit für das Zustandekommen der Osmose geeignet sind. Beispielsweise können künstlich hergestellte Membranen aus Celluloseazetat, Cellulosetriazetat, Agarazetat, Amyloseazetat, Celluloseazetatäthylcarbamat, Celluloseazetat-phthalat, Celluloseazetat-methylcarbamat, Celluloseazetat-succinat, Celluloseazetat-dimethylaminoazetat, Celluloseazetat-äthylcarbonat, Celluloseazetat-chlorazetat, Celluloseazetat-äthyloxalat, Celluloseazetat-methylsulfonat, Celluloseazetat-butylsulfonat, Celluloseäther, Celluloseazetat-propionat, Celluloseazetat-diäthylaminoazetat, Celluloseazetat-octat, Celluloseazetat-laurat, Methylcellulose, Celluloseazetat-p-toluolsulfonat, hydroxyliertem Aethylen-vinylazetat, Celluloseazetat-butyrat und aus anderen Celluloseazetatderivaten verwendet werden. Als semipermeable Membranen eignen sich auch Hydroxypropylmethylcellulose und polymere Epoxide, Copolymere aus Alkylenoxid und Alkylglycidyläther, Polyglykole oder Polymilchsäure derivate und weitere Abkömmlinge davon. Ferner können auch Mischungen, wie z.B. von an sich wasserunlöslichen Acrylaten (z.B. Copolymerisat von Acrylsäureäthylester und Methacrylsäuremethylester) verwendet werden.

Das einen Film (Membran) bildende Ueberzugmaterial kann mit Hilfe eines beliebigen Verfahrens aufgebracht werden, soweit dies zu einem kontinuierlichen Film von im wesentlichen einheitlicher Dicke führt.

Das Ueberziehen beispielsweise der Tablette (Pressling) und auch des Pellets mit einem je nach Bedarf dicken Film aus einer semipermeablen Membran kann in Wirbelschichten, in Dragierkesseln oder auch mit Hilfe des Koazervierungsverfahrens erfolgen.

Als quellbare nicht-kolloidale Hilfsstoffe (Quell- oder auch Sprengmittel) eignen sich indifferente, bei Berührung mit wässrigen Flüssigkeiten rasch quellende Stoffe, wie z.B. Alginsäure und -derivate, Ultraamidopektine, Cellulose, wie z.B. mikrokristalline oder mikrofeine Cellulose, Carboxymethylcellulose-crosslinked, Carboxymethyl-Stärke, modifizierte Stärke, Polyvinylpolypyrrolidone-crosslinked, Bentonite, Veegum, Montmorillonite, getrockneter Citruspulp, Xylane und auch kationische und anionische Austauscher, wie z.B. Cholestyramine.

Als wasserlösliche, die Osmose induzierende Trägerstoffe können beispielsweise die Magen- bzw. Darmschleimhäute nicht irritierende Substanzen, wie z.B. anorganische oder organische Salze, wie z.B. Kochsalz, Natriumhydrogenphosphat, Natriumnitrat und Natriumazetat oder auch Alkali- bzw. Erdalkalisalze der Kohlensäure, wie z.B. Natrium- oder auch Calciumcarbonat oder auch -hydrogencarbonate oder auch wasserlösliche organische Säuren, wie z.B. die Wein-, Zitronen-, oder auch Bernsteinsäure alleine oder in Kombination mit den vorgenannten Carbonaten und auch Zucker, insbesondere z.B. Mannit, Glukose, Fruktose, Lactose und Dextranverbindungen mit verschiedenem Molekulargewicht zur Erzeugung des Osmosedruckes bzw. zur Induktion der für den Quellvorgang notwendigen Osmose verwendet werden. Die Menge des Trägerstoffes kann von einem Bruchteil bis Mehrfachem der Menge des eingesetzten festen Wirkstoffes bzw. Wirkstoffgemisches varieren.

Als weitere Hilfsstoffe können gegebenenfalls auch Gleitmittel, wie z.B. Magnesiumstearat, Siliciumaerogel und insbesondere auch Talk verwendet werden.

Als fakultative Zusätze im Filmüberzug können z.B. unter anderem Pigmente, wie z.B. farbige Eisenoxyde oder Titandioxyd und/oder Geschmackstoffe, wie z.B. Süssstoffe (z.B. Saccharin, Na-Cyclamat oder Zucker) verwendet werden.

Als Formkörper (Kern) aus Wirkstoff, bzw. Wirkstoffgemischen und Hilfsstoffen kommen die in der Galenik üblichen verpressten Tabletten (Presslinge), Kapseln und Pellets in Frage, wie sie nach bekannten Verfahren hergestellt werden können. Beispielswseise kann die Herstellung der Tablettenmasse durch Mischen der Feststoffteilchen, d.h. des Wirkstoffes, bzw. Wirkstoffgemisches, Quellstoffes, der Trägerstoffe und gegebenenfalls anderer Hilfsstoffe, wie z.B. Gleitmittel und im Bedarfsfalle auch gewünschtenfalls Retardierungsmitteln erfolgen. Die Herstellung der Presslinge und Pellets kann mit den für die Herstellung von beispielsweise runden und stäbchenförmigen Presslingen bzw. Pellets bekannten Tablettierungsmaschinen und das Füllen der Kapseln mit den bekannten Kapselabfüllmaschinen erfolgen.

Als retardierende Hilfsstoffe können im wesentlichen wasserunlösliche Hilfsstoffe oder Gemische davon, wie Lipide, u.a. Fettalkohole, z.B. Cetylalkohol, Stearylalkohol und Cetostearylalkohol; Glyceride, z.B. Glycerinmonostearat oder Gemische von Mono-, Di- und Triglyceriden pflanzlicher Oele; hydrierte Oele, wie hydriertes Rizinusöl oder hydriertes Baumwollsamenöl; Wachse, z.B. Bienenwachs oder Carnaubawachs; feste Kohlenwasserstoffe, z.B. Paraffin oder Erdwachs; Fettsäuren, z.B. Stearinsäure; gewisse Cellulosederivate, z.B. Aethylcellulose oder Acetylcellulose; Polymere oder Copolymere, wie Polyalkylene, z.B. Polyäthylen, Polyvinylverbindungen, z.B. Polyvinylchlorid oder Polyvinylazetat, sowie Vinylchlorid-Vinylazetat-Copolymere und Copolymere mit Crotonsäure oder Polymere und Copolymere von Acrylaten und Methacrylaten, z.B. Copolymerisate von Acrylsäureäthylester und Methacrylsäuremethylester, verwendet werden.

Insbesondere betrifft die Erfindung eine feste oral zu verabreichende Darreichungsform, die geeignet ist, den Wirkstoff, bzw. das Wirkstoffgemisch gezielt nach einer bestimmten Zeit pH-unabhängig, d.h. unabhängig von Wasserstoff- und Hydroxylionen und auch anderer Ionen, wie z.B. Phosphationen und Enzymen im Gastrointestinaltrakt quantitativ freizugeben.

Die erfindungsgemässe Darreichungsform kann insbesondere dann verwendet werden, wenn eine Freigabe des Wirkstoffes, bzw. Wirkstoffgemisches nach mehreren gewünschten hintereinander folgenden Zeitintervallen, beispielsweise nach Ablauf einiger Stunden mehrfach erfolgen sollte. Insbesondere kann die feste pharmazeutische Darreichungsform verwendet werden, wenn die Wirkstoffabgabe jeweils nach Ablauf mehrerer gleicher Zeitintervalle, d.h. periodisch erfolgen sollte. Beispielsweise können Pellets, die nach dem Verfahren der erfindungsgemässen Darreichungsform hergestellt worden sind, jedoch eine zeitlich verschiedene und damit verschobene Freigabe aufweisen, in einer einzigen Dosierungsform, wie z.B. Gelatinekapsel verabreicht werden. Die in verschiedenen Zeitintervallen zu erfolgende Freigabe kann, wie bereits eingangs gesagt, durch die Schichtdicke der Membran, mechanische Festigkeit und Elastizität und gegebenenfalls durch die Menge und Quelleigenschaft des Quell- bzw. Sprengmittels und Osmose induzierende Eigenschaft des Trägerstoffes oder entsprechende Kohlendioxyd erzeugende Trägerstoffe gemäss beiliegender Zeichnung (A) präzise gesteuert werden. Aus der Zeichnung ist ersichtlich, dass die Freigabe des Wirkstoffes (Diclofenac-Natrium) in Uebereinstimmung mit den Angaben der Tabelle I des Beispiels 1 nach verschiedenen Zeitintervallen erfolgt.

Der hier verwendete Ausdruck "pharmazeutischer Wirkstoff" umfasst alle therapeutisch verwendbaren Mittel, wie z.B. die in der Human-bzw. Veterinärmedizin verwendbaren therapeutisch aktiven Substanzen.

Die erfindungsgemässe Darreichungsform eignet sich beispielsweise für wasserlösliche und auch wasserunlösliche feste pharmazeutische Wirkstoffe, welche anorganische oder insbesondere organische Wirksubstanzen sein können und gemäss ihrer Indikation als Analgetika, Antipyretika, Antirheumatika, Sedativa, Hypnotika, Antiepileptika, Neuraltherapeutika, Narkotika, Neuroleptanalgetika, Antihistaminika, Antihypertensiva, Antikoagulantien, Antitrombolika, Psychopharmaka, Psychoanaleptika, Chemotherapeutika, wie z.B. Antibiotika, Sulfonamide, Antitüberkulotika, (Tüberkulostatika) oder auch Chemotherapeutika gegen Tropeninfektionen, Diuretika, Spasmolytika, Kreislaufmittel, wie z.B. Sympathomimetika, Antihypertonika, Herzmittel, wie z.B. Herzglykoside und Digitaloide, parenterale Zuckertherapeutika, zentralwirkende Analeptika, Geriatrika, Tonolytika (der quer gestreiften Muskulatur), Antiparkinson-Mittel, Zytostatika, Immunsuppressiva, Tonika, Hormone und Vitamine nach B. Helwig (Moderne Arzneimittel), 1980, zu verwenden sind.

Beispielsweise können als Antibiotika Penicillin, Tetracyclin, Chlortetracyclin, Bacitrazin, Nystatin, Streptomyzin, Neomyzin, Polymizin, Gramicidin, Oxytetracyclin, Chloramphenicol, Erythromyzin, Rifampizin, Cefazolin, Cefoxitin, Cefsulodin, Cefotiam und Mefoxin und als Chemoterapeutika Sulfamethazin, Sulfamerazin, Sulfamethizol und Sulfisoxazol als feste Wirkstoffe für die erfindungsgemässe Darreichungsform verwendet werden. Ferner können z.B. als Sedativa und Hypnotika Chloral-Hydrat, Midazolam, Pentabarbital, Phenobarbital, Secobarbital, Codeine und Carbromal und als Herzglykoside und Digitaloide Digitoxin und Digoxin und als Sympathomimetika Epinephrin als feste Wirkstoffsubstanz in wasserlöslicher Form oder wasserunlöslicher Form verwendet werden.

Insbesondere können Antipyretika, Analgetika und Antirheumatika in geeigneter wasserlöslicher Form oder wasserunlöslicher Form beispielsweise Propyphenazon, Aminophenazon, Aspirin (ASA), Antipyrin, Methylnifenazin, Melaminsulfon, Sulfenazon, Phenacetin, Pentazozin, Lactophenin, Paracetamol, Chinin, Flufenaminsäure, Mefenaminsäure, Tolfenaminsäure, Meclofenaminsäure, Nifluminsäure, Clonixin oder Clonixidin, Flunixin, Ibuprofen, Suprofen, Ketoprofen, Fenoprofen, Pirprofen, Diclofenac, Ibufenac, Proctizinsäure, Naproxen, Cicloprofen, Tolmetin, Clopirac, Tiaprofensäure, Oxaprozin, Fenclozinsäure, Fentiazac, Clidanac, Fenclonac, Fenoprofen, Flurbiprofen, Carprofen, Sulindac, Cinmetacin, Fenbuten, Etodolac, Butifufen, als fester Wirkstoff in der erfindungsgemässen Darreichungsform verwendet werden.

Vorteilhaft können Psychopharmaka, wie z.B. Neuroleptika, Antidepressiva, Thymoleptika, Thymeretika und Tranquilizer in wasserlöslicher Form oder wasserunlöslicher Form wie z.B. Thioridazin, Imipramin, Desimipramin, Clomipramin, Ketimipramin, Opipramol, Amitriptylin, Nortriptylin, Reserpin, Aromazin, Chlorpromazin, Fluopromazin, Methopromazin, Trimeprazin, Diethazin, Promethazin, Aminopromazin, Mepazin, Pipamazin und Maprotilin als fester Wirkstoff in der erfindungsgemässen Darreichungsform verwendet werden.

Insbesondere können Analgetika und Antirheumatika wie z.B. Ibuprofen, Pirprofen, Ibufenac, Naproxen und Diclofenac in der erfindungsgemässen Darreichungsform verwendet werden. Besonders vorteilhaft kann als Analgetika und Antirheumatika Diclofenac in der erfindungsgemässen Darreichungsform verwendet werden.

Ferner können auch Antihypertensiva, wie z.B. Oxprenolol und Metoprolol oder auch Hypnotica, wie z.B. Midazolam als fester Wirkstoff in der Darreichungsform verwendet werden.

In den nachfolgenden nicht einschränkenden Beispielen wird die Erfindung näher erläutert. Unter Teilen, wenn nicht anders angegeben, werden immer Gewichtsteile verstanden. Temperaturen werden in Celsiusgraden angegeben.

### Beispiel 1:

Eine oral zu verabreichende Darreichungsform (Filmtablette), die 50 mg Diclofenacnatrium als Wirkstoff im gepressten Kern (Pressling) enthält und mit einer semipermeablen Membran als Film überzogen ist, welche den Wirkstoff jeweils nach einer vorbestimmten Zeit freigibt wird wie folgt hergestellt:

### a) Herstellung des Kerns:

Die Kerne nachfolgender Zusammenstellung werden unter Verwendung eines 8 mm Concavstempels (R 12 mm) in einer mit nur einem Stempel versehenen Tablettenpresse verpresst.

| Jeder verpresste Kern enthält: | |
| --- | --- |
| Diclofenacnatrium | 50 mg |
| Polyvinylpolypyrrolidon (crosslinked) | 100 mg |
| Natriumchlorid | 50 mg |
| Silicaaerogel (Aerosil 200®) | 7 mg |
| Magnesiumstearat | 3 mg |

Das Pulver für 2000 Tablettenkerne wird 20 Minuten lang in einem Taumelmischer (turbulamixer) homogen vermischt und wie oben angegeben zu Kernen à 210 mg Gesamtgewicht verpresst.

### b) Herstellung der Filmschicht:

1500 verpressste Kerne werden unter Anwendung des Wirbelschichtverfahrens im Luftstrom mit einem semipermeablen Film der nachfolgenden Zusammensetzung lackiert.

| | |
| --- | --- |
| Celluloseazetat enthaltend 32 % Acetyl | 46,5 g |
| Celluloseazetat enthaltend 39,8 % Acetyl | 48,5 g |
| Hydroxypropylmethylcellulose | 5,0 g |

Die Lackierung erfolgt mit einer organischen Lack enthaltenden Lösung, die 5 % festen Lackbestandteil in einem im wesentlichen aus 1800 ml Methylenchlorid und 200 mg Methanol bestehenden Lösungsgemisch enthält. Die Kerne werden mit Lackschichten verschiedener Stärke (d.h. verschiedenem Gewicht), beispielsweise mit annähernd 12 mg, 22 mg, 33 mg, 47 mg und 62 mg Lack überzogen und im Luftstrom in einem Wirbelschichttrockner 48 Stunden bei 40° getrocknet.

c) Bestimmung der Freigabe des Wirkstoffes:

Je 10 Filmtabletten mit 5 verschiedenen Filmstärken (mit einem Film verschiedenen Gewichtes überzogen) werden in ein Becherglas, enthaltend 200 ml entionisiertes (entsalztes) Wasser bei 37° C gegeben und die Zeit der lidartigen Oeffnung (Sprengung) der 5 Systeme bestimmt. Die Werte sind in der Tabelle II angegeben.

Tabelle I

| System | Gewicht des Lackes | Zeit der lidartigen Oeffnung (Sprengung) |
|---|---|---|
| 1 | 12 mg | 57, 62, 64, 65, 75 ($\bar{x}$ = 65; $S_{rel}$ = 9.1) |
| 2 | 22 mg | 103, 110, 127, 128, 130 ($\bar{x}$ = 120; $S_{rel}$ = 9.2) |
| 3 | 33 mg | 159, 175, 178, 180, 188 ($\bar{x}$ = 176; $S_{rel}$ = 5.4) |
| 4 | 47 mg | 201, 201, 203, 217, 222 ($\bar{x}$ = 209; $S_{rel}$ = 4.3) |
| 5 | 62 mg | 387, 390, 393, 395, 430 ($\bar{x}$ = 399; $S_{rel}$ = 3.9) |

Beispiel 2:

Ca. 5000 Kerne, die 10 mg Oxprenololsuccinat als aktiven Wirkstoff enthalten, werden verpresst und enthalten folgende Zusammensetzung.

| | |
|---|---|
| Oxprenololsuccinat | 10 mg |
| Natriumchlorid | 25 mg |
| Cholestyramin USP | 50 mg |
| Silicaaerogel | 4,5 mg |
| Magnesiumstearat | 0,5 mg |

Die Kerne der obigen Zusammensetzung mit einem Gewicht von ca. 90 mg werden unter Verwendung eines 6 mm Concavstempels in einer mit nur einem Stempel versehenen Tablettenpresse verpresst.

Ca. 1000 verspresste Kerne werden unter Anwendung des Wirbelschichtverfahrens im Luftstrom mit einem Film bestehend aus 20 mg, 25 mg und 30 mg Lack lackiert. Die Filmmembrane setzt sich zu 40 % (Gewicht) aus einem Celluloseazetat mit einem Acetylgehalt von 32 % und 55 % (Gewicht) aus einem Celluloseazetat mit einem Acetylgehalt von 39,8 % und 5 % (Gewicht) Hydroxypropylmethylcellulose zusammen.

Die Lackierung erfolgt mit einer organischen Lack enthaltenden Lösung, die 5 % festen Lackbestandteil in einem Lösungsgemisch Methylenchlorid:Methanol im Verhältnis 9:1 enthält.

Die Kerne werden mit Lackschichten verschiedener Stärke (verschiedenem Gewichts), beispielsweise mit annähernd 20, 25 und 30 mg überzogen und dann im Luftstrom in einem Wirbelschichttrockner bei 40° C 24 Stunden lang getrocknet.

Bestimmung der Freigabe des Wirkstoffes

Je 10 Filmtabletten mit 3 verschiedenen Filmstärken (mit einem Film verschiedenem Gewichts überzogen) werden in ein Becherglas, enthaltend 200 ml entionisiertes (entsalztes) Wasser bei 37° C gegeben und die Zeit der lidartigen Oeffnung (Sprengung) der 3 Systeme bestimmt.

EP 0 408 496 A2

Tabelle II

| System | Gewicht des Lackes | Zeit der lidartigen Oeffnung (Sprengung) |
|--------|--------------------|-----------------------------------------|
| 1 | 20 mg | 118, 120, 125, 130, 131, 132, 136, 140, 142 ( $\bar{x}$ = 130; $S_{rel}$ = 6.1) |
| 2 | 25 mg | 147, 158, 159, 160, 160, 166, 179, 180, 180, 184 ( $\bar{x}$ = 167; $S_{rel}$ = 7.2) |
| 3 | 30 mg | 205, 209, 212, 212, 214, 215, 219, 219, 220, 222, ( $\bar{x}$ = 215; $S_{rel}$ = 2.4) |

Beispiel 3:

Ca. 1000 Kerne, die 7 mg Midazolam (Dormicum®) als aktiven Wirkstoff enthalten, werden verpresst und enthalten folgende Zusammensetzung.

| | |
|--------------------------------------|---------|
| Midazolam | 7 mg |
| Natriumchlorid | 50 mg |
| Polyvinylpolypyrrolidon cross-linked | 100 mg |
| Silicaaerogel | 7,0 mg |
| Magnesiumstearat | 2,0 mg |

Die Kerne der obigen Zusammensetzung mit einem Gewicht von ca. 165 mg werden unter Verwendung eines 8 mm Concavstempels in einer mit nur einem Stempel versehenen Tablettenpresse verpresst.

Verpresste Kerne werden unter Anwendung des Wirbelschichtverfahrens im Luftstrom mit einem Film bestehend aus ca. 38 mg Lack lackiert. Die Filmmembrane setzt sich zu 46,5 % (Gewicht) aus einem Celluloseazetat mit einem Acetylgehalt von 32 % und 48,5 % (Gewicht) aus einem Celluloseazetat mit einem Acetylgehalt von 39,8 % und 5 % (Gewicht) Hydroxypropylmethylcellulose zusammen.

Die Lackierung erfolgt mit einer organischen Lack enthaltenden Lösung, die 5 % festen Lackbestandteil in einem Lösungsgemisch Methylenchlorid: Methanol im Verhältnis 8:2 enthält.

Die Kerne werden mit einer Lackschicht von 38 mg überzogen und dann im Luftstrom in einem Wirbelschichttrockner bei 40° C 24 Stunden lang getrocknet.

Bestimmung der Freigabe des Wirkstoffes

10 Filmtabletten werden in ein Becherglas, enthaltend 200 ml entionisiertes (entsalztes) Wasser bei 37° C gegeben und die Zeit der lidartigen Oeffnung (Sprengung) der Systeme bestimmt.

Tabelle III

| System | Gewicht des Lackes | Zeit der lidartigen Oeffnung (Sprengung) in Min. |
|--------|--------------------|---------------------------------------------------|
| 1 | 38 mg | 250, 250, 252, 260, 260, 264, 270, 272, 275, 280 ( $\bar{x}$ = 263; $S_{rel}$ = 6.8) |

**Ansprüche**

1. Feste pharmazeutische Darreichungsform, aus der der aktive Wirkstoff bzw. das Wirkstoffgemisch annähernd pH-unabhängig, d.h. unabhängig von den Wasserstoffionen- und Hydroxylionenkonzentrationen

8

und/oder anderer Ionen und auch unabhängig von Enzymen der umgebenden Körperflüssigkeit, gezielt nach einer bestimmten Zeit, d.h. nach Ablauf eines bestimmten Zeitintervalles quantitativ sofort oder kontinuierlich in Lösung gehen kann, dadurch gekennzeichnet, dass die einen Wirkstoff oder ein Wirkstoffgemisch, einen nicht-kolloidalen in Wasser quellbaren Hilfsstoff (Sprengmittel) und mindestens einen in Wasser löslichen Trägerstoff (Osmose induzierenden Stoff) und gegebenenfalls andere Hilfsstoffe enthaltende Darreichungsform mit einer semipermeablen Membran überzogen wird, die beim Eindringen von wässriger Flüssigkeit durch den Quelldruck des quellbaren nicht-kolloidalen Hilfsstoffes und durch den gleichzeitig auftretenden osmotischen Druck und gegebenenfalls intern erzeugten Gasdruck nach einer bestimmten vorgegebenen Zeit lidartig aufspringt und den Wirkstoff bzw. das Wirkstoffgemisch quantitativ freigibt.

2. Feste pharmazeutische Darreichungsform gemäss Anspruch 1, dadurch gekennzeichnet, dass die feste Darreichungsform aus einer Filmtablette (Pressling) oder einem Pellet besteht mit einem osmotischen aktiven Kern, der neben dem Wirkstoff bzw. Wirkstoffgemisch und dem quellbaren nichtkolloidalen Hilfsstoff auch mindestens einen wasserlöslichen Trägerstoff (Osmose induzierenden Stoff) und andere Hilfsstoffe, wie z.B. Gleitmittel und Retardierungsmittel enthalten kann und einer semipermeablen Membran als Filmüberzug.

3. Feste pharmazeutische Darreichungsform gemäss Anspruch 1, dadurch gekennzeichnet, dass die feste Darreichungsform aus einer Kapsel besteht, die den Wirkstoff bzw. das Wirkstoffgemisch, den quellbaren nichtkolloidalen Hilfsstoff, mindestens einen wasserlöslichen Trägerstoff und andere Hilfsstoffe pulverförmig enthält und mit einer semipermeablen Membran als Film überzogen ist.

4. Feste Darreichungsform gemäss Ansprüchen 1 und 3, dadurch gekennzeichnet, dass die feste Darreichungsform aus einer Gelatinekapsel besteht, die den Wirkstoff bzw. das Wirkstoffgemisch, den quellbaren nicht-kolloidalen Hilfsstoff, mindestens einen Trägerstoff, Gleitmittel und Retardierungsmittel pulverförmig enthält und mit einer semipermeablen Membran als Film überzogen ist.

5. Feste Darreichungsform gemäss Ansprüchen 1-4, dadurch gekennzeichnet, dass man als quellbaren nicht-kolloidalen Hilfsstoff in der festen Darreichungsform einen indifferenten, bei Berührung mit wässrigen Flüssigkeiten rasch quellenden Stoff verwendet.

6. Feste Darreichungsform gemäss Ansprüchen 1-5, dadurch gekennzeichnet, dass man als quellbaren nicht-kolloidalen Hilfsstoff in der festen ·Darreichungsform einen aus der Gruppe wie z.B. Alginsäure und -derivate, Ultraamidopektine, Cellulose, wie z.B. mikrokristalline oder mikrofeine Cellulose, Carboxymethylcellulose-crosslinked, Carboxymethyl-Stärke, modifizierte Stärke, Polyvinylpolypyrrolidone-crosslinked, Bentonite, Veegum, Montmorillonite, getrockneten Citruspulp, Xylane und auch kationische und anionische Austauscher verwendet.

7. Feste Darreichungsform gemäss Ansprüchen 1-6, dadurch gekennzeichnet, dass man als quellbaren nicht-kolloidalen Hilfsstoff in der festen Darreichungsform Polyvinylpolypyrrolidone-crosslinked oder Cholestyramine verwendet.

8. Feste Darreichungsform gemäss Ansprüchen 1-4, dadurch gekennzeichnet, dass man als Trägerstoffe (Osmose induzierende Stoffe) einen aus der Gruppe wasserlöslicher anorganischer oder organischer Salze, wie z.B. Kochsalz, Natriumhydrogenphosphat, Natriumnitrat und Natriumazetat oder auch Alkali- bzw. Erdalkalisalze der Kohlensäure, wie z.B. Alkali- bzw. Erdalkalicarbonate oder -hydrogencarbonate oder auch wasserlösliche organische Säuren, wie z.B. die Wein-, Zitronen-, oder auch Bernsteinsäure alleine oder in Kombination mit den vorgenannten Carbonaten und auch Zucker, insbesondere z.B. Mannit, Glukose, Fruktose, Lactose und Dextranverbindungen mit verschiedenem Molekulargewicht verwendet.

9. Feste Darreichungsform gemäss Ansprüchen 1-4, dadurch gekennzeichnet, dass man als semipermeable Membran als Filmüberzug in der festen Darreichungsform eine Membran verwendet, die für den Durchgang von Wasser aus vorhandener Körperflüssigkeit und nicht des gelösten Wirkstoffes geeignet ist.

10. Feste Darreichungsform gemäss Ansprüchen 1-4 und 9, dadurch gekennzeichnet, dass man als semipermeable Membran als Filmüberzug eine künstlich hergestellte Membran aus Celluloseazetat, Cellulosetriazetat, Agarazetat, Amyloseazetat, Celluloseazetat-äthylcarbamat, Celluloseazetat-phthalat, Celluloseazetat-methylcarbamat, Celluloseazetat-succinat, Celluloseazetat-dimethylaminoazetat, Celluloseazetat-äthylcarbonat, Celluloseazetat-chlorazetat, Celluloseazetat-äthyloxalat, Celluloseazetat-methylsulfonat, Celluloseazetat-butylsulfonat, Celluloseäther, Celluloseazetat-propionat, Celluloseazetat-diäthylaminoazetat, Celluloseazetat-octat, Celluloseazetat-laurat, Methylcellulose, Celluloseazetat-p-toluolsulfonat, hydroxyliertem Aethylen-vinylazetat, Celluloseazetat-butyrat, Hydroxypropylmethylcellulose aus polymeren Epoxiden, Copolymeren aus Alkylenoxid und Alkylglycidyläthern, Polyglykolen oder Polymilchsäurederivaten und aus Acrylaten (z.B. Copolymerisat von Acrylsäureäthylester und Methacrylsäuremethylester) verwendet.

11. Feste Darreichungsform gemäss Ansprüchen 1-9, dadurch gekennzeichnet, dass man als Wirkstoffe bzw. Wirkstoffgemische, feste pharmazeutische Wirkstoffe, welche anorganische oder insbesondere organische Wirkstubstanzen sein können und gemäss ihrer Indikation als Analgetika, Antipyretika, Antirheumatika, Sedativa, Hypnotika, Antiepileptika, Neuraltherapeutika, Narkotika, Neuroleptanalgetika, Antihistaminika, Antihypertensiva, Antikoagulantien, Antitrombolika, Psychopharmaka, Psychoanaleptika, Chemotherapeutika, wie z.B. Antibiotika, Sulfonamide, Antitüberkulotika, (Tüberkulostatika) oder auch Chemotherapeutika gegen Tropeninfektionen, Diuretika, Spasmolytika, Kreislaufmittel, wie z.B. Sympathomimetika, Antihypertonika, Herzmittel, wie z.B. Herzglykoside und Digitaloide, parenterale Zuckertherapeutika, zentralwirkende Analeptika, Geriatrika, Tonolytika (der quer gestreiften Muskulatur), Antiparkinson-Mittel, Zytostatika, Immunsuppressiva, Tonika, Hormone und Vitamine nach B. Helwig (Moderne Arzneimittel), 1980, einzusetzen sind, verwendet.

12. Feste pharmazeutische Darreichungsform gemäss Ansprüchen 1-9, dadurch gekennzeichnet, dass man als Wirkstoff Antibiotika, wie z.B. Penicillin, Tetracyclin, Chlortetracyclin, Bacitrazin, Nystatin, Streptomyzin, Neomyzin, Polymizin, Gramicidin, Oxytetracyclin, Chloramphenicol, Erythromyzin, Rifampizin, Cefazolin, Cefoxitin, Cefsulodin, Cefotiam und Mefoxin verwendet.

13. Feste pharmazeutische Darreichungsform gemäss Ansprüchen 1-9, dadurch gekennzeichnet, dass man als Wirkstoffe Chemotherapeutika, wie z.B. Sulfamethazin, Sulfamerazin, Sulfamethizol und Sulfisoxazol verwendet.

14. Feste pharmazeutische Darreichungsform gemäss Ansprüchen 1-9, dadurch gekennzeichnet, dass man als Wirkstoffe Sedativa und Hypnotika, wie z.B. Chloral-Hydrat, Midazolam, Pentabarbital, Phenobarbital, Secobarbital, Codeine und Carbromal verwendet.

15. Feste pharmazeutische Darreichungsform gemäss Ansprüchen 1-9, dadurch gekennzeichnet, dass man als Wirkstoffe Herzglykoside und Digitaloide, wie z.B. Digitoxin und Digoxin verwendet.

16. Feste pharmazeutische Darreichungsform gemäss Ansprüchen 1-9, dadurch gekennzeichnet, dass man als Wirkstoffe Sympathomimetika, wie z.B. Epinephrin verwendet.

17. Feste pharmazeutische Darreichungsform gemäss Ansprüchen 1-9, dadurch gekennzeichnet, dass man als Wirkstoffe Antipyretika, Analgetika und Antirheumatika, wie z.B. Propyphenazon, Aminophenazon, Aspirin (ASA), Antipyrin, Methylnifenazin, Melaminsulfon, Sulfenazon, Phenacetin, Pentazozin, Lactophenin, Paracetamol, Chinin, Flufenaminsäure, Mefenaminsäure, Tolfenaminsäure, Meclofenaminsäure, Nifluminsäure, Clonixin oder Clonixidin, Flunixin, Ibuprofen, Suprofen, Ketoprofen, Fenoprofen, Pirprofen, Diclofenac, Ibufenac, Proctizinsäure, Naproxen, Cicloprofen, Tolmetin, Clopirac, Tiaprofensäure, Oxaprozin, Fenclozinsäure, Fentiazac, Clidanac, Fenclonac, Fenoprofen, Flurbiprofen, Carprofen, Sulindac, Cinmetacin, Fenbuten, Etodolac, Butifufen verwendet.

18. Feste pharmazeutische Darreichungsform gemäss Ansprüche 1-9, dadurch gekennzeichnet, dass man als Wirkstoffe Analgetika und Antirheumatika Ibuprofen, Pirprofen, Ibufenac, Naproxen und Diclofenac verwendet.

19. Feste pharmazeutische Darreichungsform gemäss Ansprüche 1-9, dadurch gekennzeichnet, dass man Wirkstoff Diclofenac verwendet.

20. Feste pharmazeutische Darreichungsform gemäss Ansprüchen 1-9, dadurch gekennzeichnet, dass man als Wirkstoffe Neuroleptika, Antidepressiva, Thymoleptika, Thymeretika und Tranquilizer, wie z.B. Thioridazin, Imipramin, Desimipramin, Clomipramin, Ketimipramin, Opipramol, Amitriptylin, Nortriptylin, Reserpin, Aromazin, Chlorpromazin, Fluopromazin, Methopromazin, Trimeprazin, Diethazin, Promethazin, Aminopromazin, Mepazin, Pipamazin und Maprotilin verwendet.

21. Feste pharmazeutische Darreichungsform gemäss Ansprüchen 1-9, dadurch gekennzeichnet, dass man als Wirkstoffe Antihypertensiva, wie z.B. Oxprenolol oder Metoprolol verwendet.

22. Feste pharmazeutische Darreichungsform gemäss Ansprüchen 1-9, dadurch gekennzeichnet, dass man als Wirkstoff Midazolam verwendet.

23. Verfahren zur Herstellung einer festen pharmazeutischen Darreichungsform aus der der aktive Wirkstoff bzw. das Wirkstoffgemisch annährend pH-unabhängig, d.h. unabhängig von den Wasserstoffionen-und Hydroxylionenkonzentrationen und/oder anderer Ionen und auch unabhängig von Enzymen der umgebenden Flüssigkeit, gezielt nach einer bestimmten Zeit, d.h. nach Ablauf eines bestimmten Zeitintervalles quantitativ sofort oder kontinuierlich in Lösung gehen kann, dadurch gekennzeichnet, dass man den aktiven Wirkstoff, bzw. das Wirkstoffgemisch in Gegenwart eines quellbaren nicht-kolloidalen Hilfsstoffes und mindestens eines wasserlöslichen Trägerstoffes (Osmose induzierend) und gegebenenfalls anderer Hilfsstoffe, wie z.B. Gleitmittel und Retadierungsmittel zu einer pharmazeutischen Dosisform, wie z.B. Tablette oder Pellet verpresst, bzw. in einer Kapsel pulverförmig einlagert und die pharmazeutische Dosisform mit einer semipermeablen Membran als Film überzieht, die geeignet ist beim Eindringen von Wasser aus vorhandener Körperflüssigkeit durch den Quelldruck des quellbaren nicht-kolloidalen Hilfsstoffes und durch den gleichzeitig auftretenden osmotischen Druck und gegebenenfalls intern erzeugten Gasdruck nach einer bestimmten vorgegebenen Zeit lidartig aufzuspringen und den Wirkstoff bzw. das Wirkstoffgemisch quantitativ freizugeben.